# EUROPEAN PATENT SPECIFICATION

(11) **EP 2 564 886 B1**
(45) Date of publication and mention of the grant of the patent: **01.05.2019**
(21) Application number: 12182193.8
(22) Date of filing: 29.08.2012
(51) Int. Cl.: A61M 1/36, A61M 25/00

(54) **Dialysis catheter**
Dialysekatheter
Cathéter de dialyse

(30) Priority: 30.08.2011 JP 2011186775
(43) Date of publication of application: 06.03.2013
(73) Proprietor: Covidien LP, Mansfield, MA 02048 (US)
(72) Inventor: Makino, Masanori, Shizuoka 437-0004 (JP); Kanie, Nobuatsu, Shizuoka 437-0004 (JP); Akaike, Yoshimi, Shizuoka 437-0004 (JP)
(74) Representative: Gray, James

(56) References cited:
- JP-A- 2001 340 466
- US-A- 4 692 141
- US-A- 5 348 536
- US-A1- 2005 182 354
- US-A1- 2009 118 661

## Description

### BACKGROUND

### Technical Field

The present invention relates to a dialysis catheter comprising multiple lumens.

### Description of Prior Art

Medical catheters are conventionally used in fields of medical care such as surgery, treatment and diagnosis for applications in which liquid is removed from a body cavity and fluid is introduced into a body cavity at the same time. A specific example of this is an application in which blood is removed from a blood vessel and purified blood is once again introduced into the blood vessel in order to perform dialysis using a hemodialysis apparatus. Furthermore, the dialysis catheter used for this kind of application is a well known type of catheter referred to as a double-lumen catheter which is provided with two lumens formed by partitioning the inside of a catheter main body with a dividing wall See, e.g., published Japanese translation of PCT Application JP 2007-521913, and published Japanese translation of PCT Application JP 2001-340466. A dialysis catheter of this kind comprises a blood removal lumen having a blood removal hole, and a blood feed lumen having a blood feed hole, among other things. Blood is therefore removed by way of the blood removal hole and the blood removal lumen, while at the same time purified blood is introduced into the blood vessel by way of the blood feed lumen and the blood feed hole. Furthermore, a different type of dialysis catheter which is also well known is referred to as a triple-lumen catheter and is provided with a transfusion lumen in addition to a blood removal lumen and a blood feed lumen. See e.g., published Japanese translation of PCT Application JP 2007-502678.

Dialysis catheters are broadly divided into end-hole type catheters and side-hole type catheters according to the difference in the positions where the blood removal hole and blood feed hole are formed. With end-hole type catheters, the blood removal hole and blood feed hole are both disposed in the same position at the tip end of the catheter. Both holes constitute tip-end holes which open towards the catheter long-axis direction. With side-hole type catheters, on the other hand, the blood feed hole is disposed at the tip end of the catheter while the blood removal hole is disposed at a position further towards the base end. Of these two holes, at least the blood removal hole constitutes a side hole which opens towards the radial direction of the catheter. Incidentally, although the resistance to insertion into a blood vessel of end-hole type catheters is greater and the ease of insertion can hardly be considered good, the patency is good and therefore the blood replacement efficiency is good. Side-hole type catheters are relatively easy to insert, but the blood removal hole is likely to be sucked against the blood vessel wall, and therefore the patency is poor and it is not possible to ensure an adequate flow rate of blood, which means that the blood replacement efficiency can hardly be considered good. In this regard, efforts have been made to ensure an adequate flow rate of blood in conventional side-hole type dialysis catheters by taking measures such as providing multiple blood removal holes, for example.

However, when multiple blood removal holes are to be provided in a conventional side-hole type dialysis catheter, the diameter of each of the blood removal holes is inevitably reduced in view of the relationship between space and strength etc. However, if this structure is adopted, the blood removal holes may be closed off as they suck against the blood vessel wall, in which case the patency deteriorates and it is difficult to ensure an adequate flow rate of blood. There is still therefore room for improvement in relation to the blood replacement efficiency. In addition, depending on the size and shape of the blood removal holes there may also be greater resistance to insertion and withdrawal, something which is contrary to the intended aim.

Furthermore, a closure member provided at the tip-end opening of the blood removal lumen closes off said opening in the abovementioned conventional dialysis catheter, in order to prevent blood from accumulating at the tip end of the blood removal lumen. Furthermore, this kind of closure member has a shape in which the tip-end side is inclined with a reduced diameter which serves to reduce the insertion resistance, and this is advantageous in terms of improving the ease of insertion. The base-end side of the closure member extends close to the position on the blood removal lumen where the blood removal hole is formed, but there are problems in that blood accumulates in that region and the flow of blood is disturbed, among other things. It would therefore be desirable to provide measures for preventing the accumulation of blood etc., which causes the formation of blood clots and adhesion.

US 2009/118661 A1 describes a double lumen dialysis catheter.

The present invention has been devised in view of the issues mentioned above, and the object thereof lies in providing a dialysis catheter which is superior in terms of both ease of insertion/withdrawal and blood replacement efficiency, and which also makes it possible to prevent the accumulation of blood etc., which causes the formation of blood clots and adhesion.

### SUMMARY

In one embodiment there is provided, a dialysis catheter as defined by claim 1.

The side hole is therefore a long hole having a relatively large diameter which extends in the catheter long-axis direction, so the cross-sectional area of the flow path can be increased. Furthermore, when this kind of side hole is used as a blood removal hole, there is less likelihood of suction against the blood vessel wall causing closure. This means that there is good patency and it is possible to ensure an adequate flow rate of blood, and the blood replacement efficiency can be improved. Moreover, when viewed from the first direction, the angle formed by the tip-end opening edge of the side hole and the catheter long-axis direction, and the angle formed by the base-end opening edge of the side hole and the catheter long-axis direction are both small angles. Consequently, the two opening edges of the side hole are unlikely to catch on the blood vessel when the catheter is inserted or withdrawn, so the catheter can be inserted and withdrawn with little resistance. This means that the catheter can be inserted and withdrawn more easily. In addition, when viewed from the first direction, the base-end surface of the closure member is shaped to follow the shape of the tip-end opening surface of the side hole, so it is possible to prevent adhesion and formation of blood clots, without blood accumulating in that region or disturbing the flow of blood.

The side hole may have a shape, when viewed from the first direction, in which the side wall forming the second lumen has been cut away in the shape of an arc, and the base-end surface of the closure member forms a concave curved surface.

The two opening edges of the side hole may therefore be even less likely to catch on the blood vessel when the catheter is
inserted or withdrawn, so the catheter can be inserted and withdrawn more easily. Furthermore, the base end surface of the closure member may be a concave curved surface, so the blood can smoothly flow along the base end surface without blood accumulating or the flow of blood being disturbed.

The catheter main body may have a hardness gradient so as to become softer from the base-end side towards the tip-end side.

The tip-end side of the catheter main body may therefore be relatively soft, so it is possible to reduce irritation of the blood vessel wall, even without special provision of a separate member made of a low-hardness material, for example, at the very tip end. Furthermore, the base-end side of the catheter main body may have a certain degree of hardness, so the catheter insertion part is unlikely to buckle, there may be excellent torque transmission to the tip-end side, and it may be possible to maintain suitable operability.

The side wall of the catheter main body forming the first lumen and the side wall forming the second lumen may have a two-layer structure comprising a soft resin layer which is relatively flexible and a hard resin layer which is relatively rigid, and the proportion occupied by the soft resin layer increases from the base-end side towards the tip-end side, while a dividing wall which partitions the catheter main body into the first lumen and the second lumen comprises only the hard resin layer.

The abovementioned two-layer structure may be employed to produce a catheter main body having a hardness gradient with relative ease. Furthermore, even though the tip-end side of the catheter main body may be softer overall than the base-end side, the dividing wall may be rigid, so it is possible to form a large-diameter side hole while the required strength is maintained.

A double-lumen catheter may comprise a blood feed lumen serving as the first lumen and a blood removal lumen serving as the second lumen. The tip-end hole may be a blood feed hole, the side hole may be a blood removal hole, the tip-end side of the closure member may close off the tip-end opening of the blood removal lumen, and the base-end side thereof may seal the tip-end region of the blood removal hole.

Blood may therefore be removed by way of the blood removal hole and the blood removal lumen, while at the same time purified blood can be introduced into the blood vessel by way of the blood feed lumen and the blood feed hole. Furthermore, the side hole constituting the blood removal hole may have a large flow path cross-sectional area, so it may be possible to provide a double-lumen catheter in which the catheter is unlikely to be sucked against the blood vessel wall causing closure and so the patency may be excellent.

A triple-lumen catheter may comprise a transfusion lumen serving as the first lumen, a blood feed lumen serving as the second lumen and a blood removal lumen serving as the second lumen; and the tip-end hole may be a transfusion hole. The side hole positioned at the tip-end side may be a blood feed hole, and the side hole positioned at the base-end side may be a blood removal hole. The tip-end side of the closure member may close off the tip-end opening of the blood feed lumen and the tip-end opening of the blood removal lumen, and the base-end side thereof may seal the tip-end region of the blood feed hole and the tip-end region of the blood removal hole.

Blood may therefore be removed by way of the side hole positioned on the base-end side, which is the blood removal hole, and the blood removal lumen, while at the same time purified blood can be introduced into the blood vessel by way of the blood feed lumen and the side hole positioned on the tip-end side, which is the blood feed hole. If necessary, it may also be possible to introduce a
liquid such as a medicament into the blood vessel by way of the transfusion lumen and the tip-end hole, which is the transfusion hole. In addition, the two side holes constituting the blood removal hole and the blood feed hole may both have a large flow path cross-sectional area, so it may be possible to provide a triple-lumen catheter in which the catheter is unlikely to be sucked against the blood vessel wall causing closure and in which the patency is excellent. It should be noted that the two tip-end openings are respectively closed off by a shared closure member, which means that it is possible to reduce the number of components compared with when these openings are separately closed off, thereby simplifying the structure.

As described above in detail, the present invention makes it possible to provide a dialysis catheter which is superior in terms of both ease of insertion/withdrawal and blood replacement efficiency, and which also makes it possible to prevent the accumulation of blood etc., which causes the formation of blood clots and adhesion.

### BRIEF DESCRIPTION OF THE DRAWINGS

Figure 1 is a side view, with partial omissions, showing a dialysis catheter (double-lumen catheter) according to a first embodiment of the present invention;
Figure 2 is an oblique view showing the tip-end structure of the dialysis catheter according to the first embodiment shown in Figure 1;
Figure 3 is a plan view showing the tip-end structure of the dialysis catheter according to the first embodiment shown in Figure 1;
Figure 4 is a side view of the tip-end structure of the dialysis catheter according to the first embodiment shown in Figure 1;
Figure 5 is a view in cross section along the section line A-A in Figure 3;
Figure 6 (a) is a plan view of the closure member of the catheter shown in Figure 1, and (b) is a side view of the closure member shown in Figure 6(a);
Figure 7 (a) is a front view showing the tip-end structure of the dialysis catheter shown in Figure 1; (b) is a view in cross section along the section line B-B in Figure 4, (c) is a view in cross section along the section line C-C in Figure 4, (d) is a view in cross section along the section line D-D in Figure 4, and (e) is a view in cross section of the base end of the catheter main body of the catheter shown in Figure 1;
Figure 8 is a side view of the tip-end structure of a dialysis catheter according to a comparative example;
Figure 9 is a side view of the tip-end structure of a dialysis catheter according to another comparative example;
Figure 10 is a side view of the tip-end structure of a dialysis catheter according to another comparative example;
Figure 11 is a plan view showing a dialysis catheter (triple-lumen catheter) according to a second embodiment of the present invention;
Figure 12 is a bottom view showing the tip-end structure of the dialysis catheter shown in Figure 11;
Figure 13 is a side view of the tip-end structure of the dialysis catheter shown in Figure 11;
Figure 14 is a view in cross section along the section line E-E in Figure 13;
Figure 15 (a) is a plan view showing the closure member of the catheter shown in Figure 11; (b) is a bottom view showing the closure member of the catheter shown in Figure 11; and (c) is a side view showing the closure member of the catheter shown in Figure 11; and
Figure 16 is a side view of the tip-end structure of a dialysis catheter according to another embodiment.

### DETAILED DESCRIPTION OF EMBODIMENTS

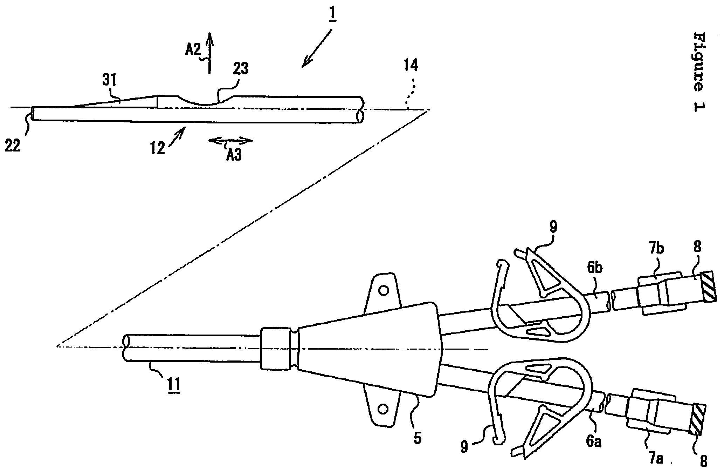

A dialysis catheter 1 (double-lumen catheter) according to a first embodiment of the present invention will be described in detail below in conjunction with Figures 1 to 7.

As shown in Figure 1 etc., the dialysis catheter 1 according to this embodiment is provided with a catheter main body (shaft) 11 of circular cross section which is made of a resilient, flexible resin material formed to be elongate. A connecting part 5 comprising two connecting tubes 6a, 6b is joined as a single piece to the base end of the catheter main body 11. The connecting tubes 6a, 6b respectively communicate with the lumens of the catheter main body 11. Luer adapters 7a, 7b to which a dialysis circuit etc. is connected are formed as a single piece at the tip ends of the connecting tubes 6a, 6b. It should be noted that the connecting tubes 6a, 6b and the luer adapters 7a, 7b are formed by separate elements, and they may be fixed to one another by means of bonding or the like.

A thread 8 is formed on the outer peripheral surface of each of the openings of the connecting tubes 6a, 6b. Furthermore, a clamp 9 for closing off the flow path is provided part way along each of the connecting tubes 6a, 6b. In the case of a dialysis catheter which is to be left indwelling for a long period of time, a cuff made of synthetic fibres may be provided at a position close to the base end of the catheter main body 11 in order to prevent the ingress of bacteria.

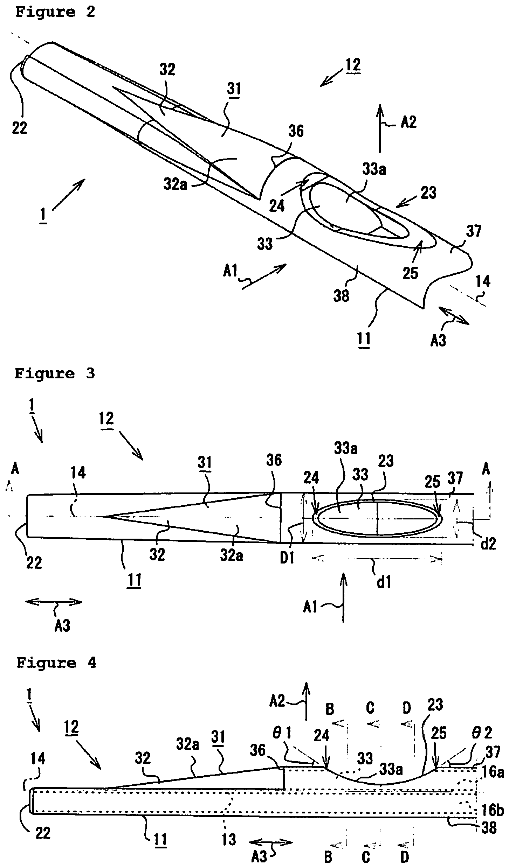

As shown in Figures 4, 5 and 7 etc., the catheter main body 11 which forms the dialysis catheter 1 comprises a dividing wall 13 which extends in the direction of the catheter long axis 14 (denoted by A3 in the figures). A blood removal lumen 16a which has a semicircular shape in cross section (the second lumen in this embodiment) is then defined by the dividing wall 13 and a side wall 37 of the catheter main body 11 forming the second lumen. Furthermore, a blood feed lumen 16b which has a semicircular shape in cross section (the first lumen in this embodiment) is defined by the dividing wall 13 and a side wall 38 of the catheter main body 11 forming the first lumen. In this embodiment, the upper part in Figures 4, 5 and 7 is used as the blood removal lumen 16a, and the lower part is used as the blood feed lumen 16b.

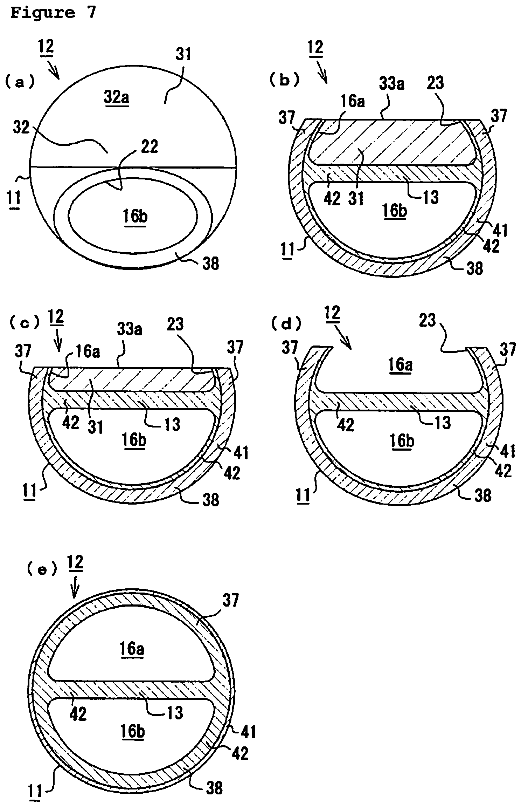

Figure 7(e) is a view in cross section of the base end of the catheter main body 11, and Figures 7(b) - (d) are all views in cross section of the tip end of the catheter main body 11. The catheter main body may be formed using a resin material of varying hardness. Specifically, the side wall 38 of the catheter main body 11 forming the first lumen and the side wall 37 forming the second lumen have a two-layer structure comprising a soft resin layer 41 which is relatively flexible and a hard resin layer 42 which is relatively rigid. The soft resin layer 41 is disposed as the outer layer and the hard resin layer 42 is disposed as the inner layer. The catheter main body 11 which has a two-layer structure of this type may be produced by extruding the soft resin layer 41 and the hard resin layer 42 at the same time by means of extrusion moulding in order to mould a single piece. The soft resin layer 41 and the hard resin layer 42 are therefore bonded together whether or not an adhesive layer is interposed. It should be noted that in this embodiment, the soft resin layer 41 and the hard resin layer 42 are each formed using the same type of resin having different hardness. More specifically, polyurethane resin having a Shore hardness of the order of A85 is used for the soft resin layer 41, and polyurethane resin having a Shore hardness of the order of D65 is used for the hard resin layer 42.

With regard to the side wall 38 forming the first lumen and the side wall 37 forming the second lumen which have the two-layer structure, the soft resin layer 41 is formed to be thin and the hard resin layer 42 is formed to be thick at the base end of the catheter main body 11 (see Figure 7(e)). That is, the soft resin layer 41 accounts for a small proportion. On the other hand, the soft resin layer 41 is formed to be thick and the hard resin layer 42 is formed to be thin at the tip end of the catheter main body 11 (see Figures 7(b) - (d)). That is, the soft resin layer 41 accounts for a large proportion. The proportion of the side wall 38 forming the first lumen and the side wall 37 forming the second lumen which is occupied by the soft resin layer 41 therefore increases from the base end of the catheter main body 11 towards the tip end. Meanwhile, the dividing wall 13 which partitions the first lumen 16b and second lumen 16a of the catheter main body 11 comprises the hard resin layer 42 alone. As a result, the catheter main body 11 of this dialysis catheter 1 has a hardness gradient so as to become softer from the base-end side towards the tip-end side.

As shown in Figures 2 - 5, the blood feed lumen 16b and the blood removal lumen 16a open at different positions on the catheter main body 11. The blood feed lumen 16b extends up to the tip end of the catheter main body 11. On the other hand, the blood removal lumen 16a does not extend up to the tip end of the catheter main body 11, and it is partially cut away. This means that the tip end structure 12 of the catheter main body 11 has a difference in level.

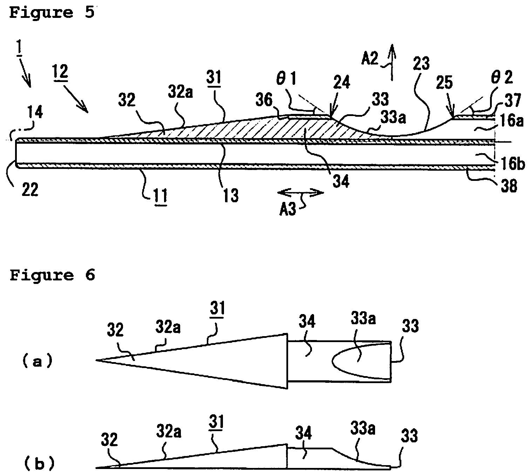

The blood feed lumen 16b communicates with a blood feed hole 22 (tip-end hole) which opens at the tip end of the catheter main body 11. The blood feed hole 22 opens towards the catheter long-axis direction A3. Meanwhile, a closure member 31 which may be classed as a protector is fixedly disposed at the difference in level of the tip-end structure 12. The closure member 31 shown in Figure 6 is a synthetic resin member formed as a separate element from the catheter main body 11, and in this embodiment, it is formed from a resin material of similar softness to the soft resin layer 41. In this case, the same resin material as that of the catheter main body 11 is preferably selected as the resin material forming the closure member 31 from the point of view of compatibility with the catheter main body 11. Polyurethane resin is selected for the soft resin layer 41 and the hard resin layer 42 in this mode of embodiment, and therefore a closure member 31 made of polyurethane resin should likewise be selected. A tip-end side 32 of the closure member 31 has a shape with a tapered surface 32a which is inclined with a reduced diameter towards the tip end. A tip-end opening 36 of the blood removal lumen 16a is closed off by insertion and welding of an insertion part 34 of the closure member 31 therein. Moreover, the shape with a tapered surface 32a which is inclined with a reduced diameter towards the tip end offers the following advantages. Namely, when the dialysis catheter 1 is inserted into a blood vessel, the blood vessel can be gradually and smoothly expanded along the tapered surface 32a, which makes insertion easier.

A blood removal hole 23 (side hole) is formed at a position (point where the side hole is formed) on the blood removal lumen 16a further towards the base end than the tip-end opening 36. The blood removal hole 23 opens towards the direction A2 orthogonal to the catheter long axis 14 and communicates with the blood removal lumen 16a.

The blood removal hole 23 serving as the side hole is a long hole extending in the catheter long-axis direction A3 which is formed by cutting away the side wall 37 forming the second lumen at the point where the side hole is formed. When the point where the side hole is formed is viewed from the opening direction A2 of the blood removal hole 23, the blood removal hole 23 exhibits an elliptical shape.

Here, as shown in Figure 3, the outer diameter D1 at the point where the side hole is formed in the catheter main body 11 is taken as a reference. Furthermore, the major axis of the blood removal hole 23 is taken as d1 and the minor axis is taken as d2. In this case, the blood removal hole 23 should be formed in such a way that the major axis d1 is at least 1.5 times the outer diameter D1, and the minor axis d2 is between 0.5 and 0.9 times the outer diameter D1.

This is because there is a risk of the flow path cross-sectional area being inadequate if the major axis d1 is less than 1.5 times the outer diameter D1. It is also because there is a risk that it will not be possible to sufficiently reduce the possibility of suction of the blood vessel wall and closure of the hole. However, the major axis d1 is preferably no more than four times the outer diameter D1 from the point of view of maintaining the strength of the catheter main body 11. In this mode of embodiment, the major axis d1 is set at around twice the outer diameter D1.

This is also because there is a risk of the flow path cross-sectional area being inadequate if the minor axis d2 is less than 0.5 times the outer diameter D1. It is also because there is a risk that it will not be possible to sufficiently reduce the possibility of suction of the blood vessel wall and closure of the hole. It is also because the side wall 37 forming the second lumen is virtually eliminated at the point where the side hole is formed if, conversely, the minor axis d2 is greater than 0.9 times the outer diameter D1, and there is a risk that it will not be possible to maintain the strength of the catheter main body 11. In this embodiment, the minor axis d2 is set at around 0.8 times the outer diameter D1 so that part of the side wall 37 forming the second lumen remains at the point where the side hole is formed (see Figures 3 and 7).

As shown in Figures 2 - 5, the direction orthogonal to the opening direction A2 of the blood removal hole 23 and orthogonal to the catheter long axis 14 direction A3 is defined as a first direction A1 for expediency. When the point where the side hole is formed is viewed from the first direction A1, the angle θ1 formed by the tip-end opening edge 24 of the blood removal hole 23 and the catheter long-axis direction A3 is no greater than 35°.

It will be understood that the angle θ1 is the angle formed by the tip-end opening edge 24 of the blood removal hole 23 and the outer surface of the side wall 37 forming the second lumen at the point where the side hole is formed. Furthermore, the angle θ2 formed by the base-end opening edge 25 of the blood removal hole 23 and the catheter long-axis direction A3 is also no greater than 35°. It will be understood that the angle θ2 is the angle formed by the base-end opening edge 25 of the blood removal hole 23 and the outer surface of the side wall 37 forming the second lumen at the point where the side hole is formed. In this embodiment, these angles θ1, θ2 are equal, both being set at approximately 33° (see Figures 4 and 5).

As shown in Figures 2, 4 and 5, a base-end side 33 of the closure member 31 extends to the position of the blood removal hole 23 and seals the tip-end region of the blood removal hole 23. The base-end side 33 of the closure member 31 has a shape in which a base-end surface 33a follows the shape of the tip-end opening surface of the blood removal hole 23, when viewed from the first direction A1. Specifically, in this embodiment, the blood removal hole 23 has a shape in which the side wall 37 forming the second lumen is cut away in the shape of an arc, when viewed from the first direction A1. The base-end surface 33a of the closure member 31 therefore has a concave curved surface which follows the shape of the arc.

The dialysis catheter 1 having the structure described above may be produced in the following manner, for example. The catheter main body 11 having the two-layer structure in which the side hole has not yet been formed is prepared first of all, and then the closure member 31 is placed at the difference in level while the insertion part 34 is inserted into the tip-end opening 36. The closure member 31 is then welded to the catheter main body 11 by heating to a prescribed temperature. After this, the blood removal hole 23 serving as the side hole is formed by punching the point of the catheter main body 11 where the side hole is formed into the shape of an arc, the same as the base-end side 33 of the closure member 31, and the base-end surface 33a is shaped accordingly. Alternatively, the catheter main body 11 having a two-layer structure in which the side hole has already been formed is prepared, and then the closure member 31 is placed at the difference in level while the insertion part 34 is inserted into the tip-end opening 36. The closure member 31 is then welded to the catheter main body 11 by heating to a prescribed temperature. After this, the base-end side 33 of the closure member 31 which can be seen from the blood removal hole 23 serving as the side hole is processed by means of punching, grinding, laser irradiation or stamping etc. in order to produce the base-end surface 33a which follows the shape of the blood removal hole 23. The former method is preferred to the latter because it enables the base-end surface 33a to be processed to the required shape with relative ease.

The method for using the dialysis catheter 1 of this embodiment will be described next. A guidewire (not depicted) is first of all inserted into the blood vessel by the usual method, after which the dialysis catheter 1 is inserted along the guidewire. In this process, the two lumens 16a, 16b inside the dialysis catheter 1 are filled with hepanarized saline. Once it has been confirmed that the dialysis catheter 1 is indwelling at the intended location, the guidewire is withdrawn. The air inside the blood feed-side lumen 16b is expelled by the usual method, after which said lumen is flushed with physiological saline or hepanarized saline. It should be noted that the same operation is also carried out for the blood removal-side lumen 16a.

The base end of the dialysis catheter 1 is then securely connected to an extracorporeal circulation circuit (that is, a dialysis circuit) and extracorporeal circulation is started. Specifically, the luer adapter 7a of the connecting tube 6a is connected to a blood removal-side port of the dialysis circuit, and the luer adapter 7b of the connecting tube 6b is connected to a blood feed-side port of the dialysis circuit. Once the connections have been made in such an order, the clamps 9 are unlocked in order to release the closure of the flow paths. When this happens, venous blood is removed by way of the blood removal lumen 16a which has the blood removal hole 23 in the tip-end region thereof. At the same time, purified blood, that is, blood from which impurities etc. have been removed, is introduced into the blood vessel by way of the blood feed lumen 16b which has the blood feed hole 22 at the tip end thereof.

Here, the blood removal hole 23 according to this embodiment is a long hole having a relatively large diameter which extends in the catheter long-axis direction A3, and therefore it has a large flow path cross-sectional area. This means that when blood is removed, it is unlikely that the blood vessel wall will be sucked against the blood removal hole 23 which would close it off, and it is possible to draw an adequate flow rate of blood into the blood removal lumen 16a. In this process, the blood which is introduced from the blood vessel at the location of the blood removal hole 23 is able to flow while being smoothly guided along the base-end surface 33a which is a concave curved surface. That is, the blood flow is streamlined so to speak at the location of the blood removal hole 23, thereby preventing accumulation of blood and disturbance of the flow of blood.

Once extracorporeal circulation has been completed, the inside of the blood removal lumen 16a is flushed with physiological saline or hepanarized saline, after which a heparin lock is applied. Moreover, the same operation is carried out for the blood feed lumen 16b.

When the dialysis catheter 1 which is not in use is to be employed for the subsequent extracorporeal circulation, the insides of the lumens 16a, 16b are flushed with physiological saline or hepanarized saline beforehand. It is then confirmed that an adequate flow rate of blood can be ensured. If an adequate flow rate of blood cannot be ensured and there are concerns about a reduction in dialysis efficiency, the indwelling position of the tip end of the catheter is moved and a suitable position is selected. Alternatively, the dialysis catheter 1 may be connected in reverse to an extracorporeal circulation circuit, and extracorporeal circulation may be carried out. That is to say, the lumen 16a which was on the blood-removal side is used as the blood-feed side, and the lumen 16b which was on the blood-feed side is used as the blood-removal side.

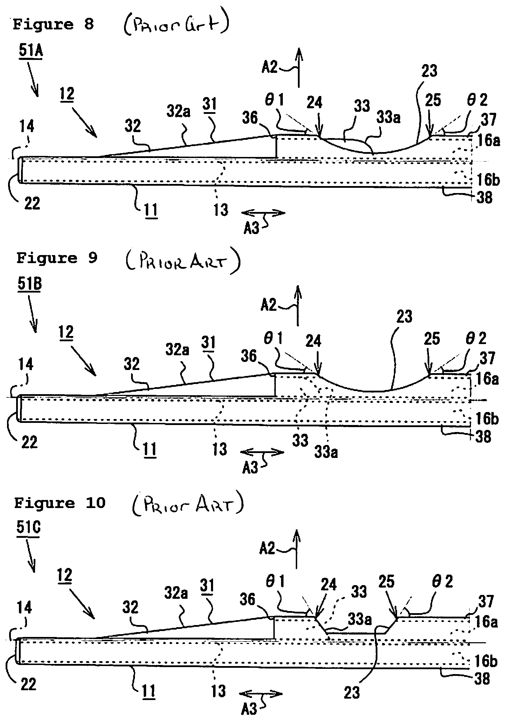

This embodiment can therefore achieve the following advantages.
(1) With the dialysis catheter 1 according to this embodiment, the blood removal hole 23, which is the side hole, is a long hole having a relatively large diameter which extends in the catheter long-axis direction A3, and the flow path cross-sectional area of the blood removal hole 23 can be increased. Furthermore, with this kind of blood removal hole 23, the blood vessel wall is unlikely to be sucked against the hole, which would close it off. The patency is therefore good and it is possible to ensure an adequate flow rate of blood, and the blood replacement efficiency can be improved.
(2) With the dialysis catheter 1 according to this embodiment, when viewed from the first direction A1, the base-end surface 33a of the closure member 31 is shaped to follow the shape of the tip-end opening surface of the blood removal hole 23. This means that blood does not accumulate and the flow of blood is not disturbed in that region, so the formation of blood clots and adhesion can be prevented. Incidentally, with the dialysis catheter 51A according to the comparative example shown in Figure 8, the base-end side 33 of the closure member 31 has a convex surface which projects, and the base-end surface 33a does not follow the shape of the tip-end opening surface of the blood removal hole 23. When that kind of convex base-end surface 33a is present, the flow of blood is likely to be disturbed in the region of the blood removal hole 23. Furthermore, with the dialysis catheter 51B according to the comparative example shown in Figure 9, conversely, the base-end side 33 of the closure member 31 is pulled inwards, in which case the base-end surface 33a does not follow the shape of the tip-end opening surface of the blood removal hole 23 either. That is to say, in this comparative example, a cavity is formed at the tip end of the blood removal hole 23, and blood is likely to accumulate and the flow of blood is likely to be disturbed in that region. This means that blood clots are likely to form and adhesion is likely to occur. This mode of embodiment makes it possible to resolve such a problem.
(3) With the dialysis catheter 1 according to this embodiment, the angle θ1 formed by the tip-end opening edge 24 of the blood removal hole 23 and the catheter long-axis direction A3, when viewed from the first direction A1, and the angle θ2 formed by the base-end opening edge 25 of the blood removal hole 23 and the catheter long-axis direction A3, when viewed from the first direction A1, are both small angles. This means that the two opening edges 24, 25 of the blood removal hole 23 are unlikely to catch on the blood vessel when the dialysis catheter 1 is inserted or withdrawn. The dialysis catheter 1 can therefore be inserted and withdrawn with low resistance. As a result it is possible to improve the ease of insertion/withdrawal of the catheter. Incidentally, with the dialysis catheter 51C of the comparative example shown in Figure 10, when viewed from the first direction A1, the blood removal hole 23 has a shape which has been cut away into the shape of an inverted trapezoid rather than an arc. The angles θ1 and θ2 are therefore somewhat larger than 35° at approximately 60°. Consequently, the opening edges 24, 25 of the blood removal hole 23 are likely to catch on the blood vessel when the dialysis catheter 1 is inserted or withdrawn, and there is a large amount of resistance during insertion and withdrawal. This embodiment makes it possible to resolve such a problem.
(4) The catheter main body 11 according to this embodiment has a hardness gradient so as to become softer from the base-end side towards the tip-end side. The tip-end side of the catheter main body 11 is therefore relatively soft, so it is possible to reduce irritation of the blood vessel wall, even without special provision of a separate member made of a low-hardness material, for example, at the very tip end. Furthermore, the base-end side of the catheter main body 11 has a certain degree of hardness, so the catheter insertion part is unlikely to buckle, there is excellent torque transmission to the tip-end side, and it is possible to maintain suitable operability.
(5) With the dialysis catheter 1 according to this embodiment, the side wall 38 forming the first lumen of the catheter main body 11 and the side wall 37 forming the second lumen have a two-layer structure comprising the soft resin layer 41 which is relatively flexible and the hard resin layer 42 which is relatively rigid. Furthermore, the proportion occupied by the soft resin layer 41 increases from the base end towards the tip end of the side wall 38 forming the first lumen and the side wall 37 forming the second lumen. On the other hand, the dividing wall 13 which partitions the first lumen 16b and the second lumen 16a of the catheter main body 11 comprises only the hard resin layer 42. This kind of two-layer structure is employed, and as a result it is therefore possible to produce the catheter main body 11 having a hardness gradient with relative ease. Furthermore, even though the tip-end side of the catheter main body 11 is softer overall than the base-end side, the dividing wall 13 is rigid, so it is possible to form the large-diameter blood removal hole 23 while the required strength is maintained.
(6) With the dialysis catheter 1 according to this embodiment, the tip end of the catheter main body 11 is relatively flexible, so a tip-end protective tip made of a material of low hardness may be omitted. This reduces the risk of detachment etc. of the member, while also making it possible to avoid increasing the number of components.

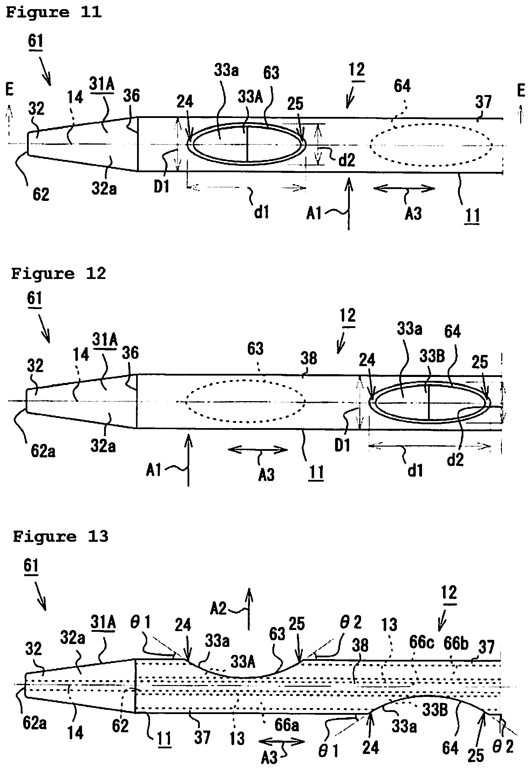

A dialysis catheter 61 (triple-lumen catheter) according to a second embodiment will be described in detail below in conjunction with Figures 11 to 15. Here, the differences with the first embodiment will chiefly be described, with common members being numbered in the same way and not being described again.

As shown in Figures 11 - 14 etc., the catheter main body 11 which forms the dialysis catheter 61 comprises the dividing wall 13 which extends in the catheter long-axis direction A3. Three lumens in the catheter main body 11 are defined by the side wall 37 forming the second lumen, the dividing wall 13 and the side wall 38 forming the first lumen. That is to say, this dialysis catheter 61 comprises three lumens, namely a transfusion lumen 66c serving as a first lumen, a blood feed lumen 66b serving as a second lumen, and a blood removal lumen 66a serving as a second lumen.

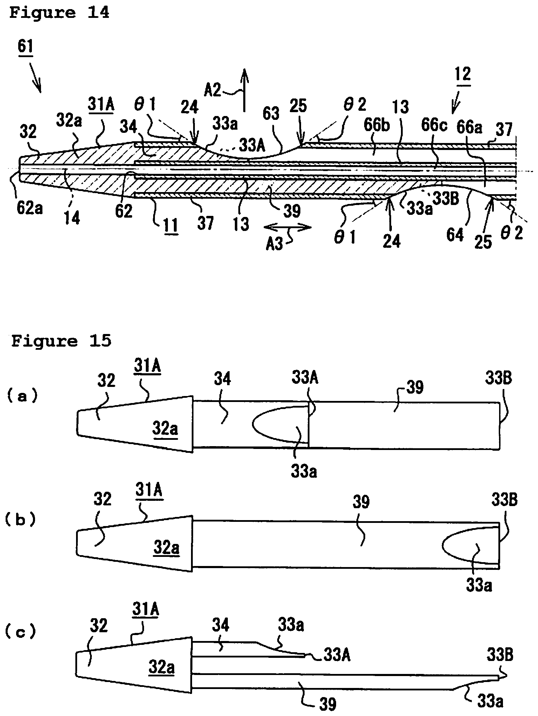

As shown in Figures 13 and 14, the transfusion lumen 66c communicates with a transfusion hole 62 (tip-end hole) which opens at the tip end of the catheter main body 11. The transfusion hole 62 opens towards the catheter long-axis direction A3. In addition, the transfusion hole 62 is placed in communication with a central hole 62a formed running through the centre of a closure member 31A. The opening in this central hole 62a forms the actual transfusion hole.

As shown in Figures 11 and 14, a blood feed hole 63, which is one of the two side holes and lies at the tip-end side, is formed at a position (point where the side hole is formed) which is slightly closer to the base end than the tip-end opening of the blood feed lumen 66b. The blood feed hole 63 opens towards the direction A2 orthogonal to the catheter long axis 14 and communicates with the blood feed lumen 66b. It should be noted that the blood feed hole 63 in this mode of embodiment has the same size and shape as that of the first embodiment.

As shown in Figures 12 and 14, a blood removal hole 64, which is one of the two side holes and lies at the base-end side, is formed at a position (point where the side hole is formed) which is closer to the base end than the tip-end opening of the blood removal lumen 66a. The blood removal hole 64 opens towards the direction A2 orthogonal to the catheter long axis 14 and communicates with the blood removal lumen 66a. It should be noted that the blood removal hole 64 in this mode of embodiment has the same size and shape as that of the first mode of embodiment.

The tip-end side 32 of the closure member 31A of this embodiment shown in Figure 15 has a shape comprising a tapered surface 32a which is inclined with a reduced diameter towards the tip end. This closure member 31A comprises two insertion parts 34, 37. The shorter insertion part 34 is the section which is inserted into the tip-end opening of the blood feed lumen 66b, and has a base end surface 33a which is a curved concave surface at the base-end part 33A thereof. The longer insertion part 37 is the section which is inserted into the tip-end opening of the blood removal lumen 66a, and has a base-end surface 33a which is a curved concave surface at the base-end part 33B thereof. These tip-end openings are sealed by inserting and welding the insertion part 34 in the tip-end opening of the blood feed lumen 66b and inserting and welding the insertion part 37 in the tip-end opening of the blood removal lumen 66a.

With the dialysis catheter 61 according to this embodiment having the structure described above, blood can be removed by way of the blood removal hole 64, which is the side hole positioned on the base-end side, and the blood removal lumen 66a. At the same time, purified blood can be introduced into the blood vessel by way of the blood feed lumen 66b and the blood feed hole 63, which is the side hole positioned on the tip-end side. Furthermore, a liquid such as a medicament can also be introduced if required, by way of the transfusion lumen 66c and the transfusion hole 62, which is the tip-end hole. In this case, the blood removal hole 64 and the blood feed hole 63, which are the two side holes, both have a large flow path cross-sectional area, and therefore the blood vessel wall is less likely to be sucked against the holes, which would close them off. The patency is therefore good and it is possible to ensure an adequate flow rate of blood, as a result of which it is possible to provide a triple-lumen catheter which has excellent blood replacement efficiency. The dialysis catheter 61 according to this embodiment naturally has advantages in that it can also be inserted/withdrawn with a great deal of ease, and also makes it possible to prevent accumulation of blood etc., which causes the formation of blood clots and adhesion.

Furthermore, the dialysis catheter 61 according to this embodiment has a structure in which two tip-end openings are each closed off by one shared closure member 31A, so to speak. Therefore it is possible to reliably reduce the number of components and to simplify the structure compared with when these openings are closed off by separate members.

In addition, with the dialysis catheter 61, the closure member 31A also serves as a tip-end protective tip. This means that it is possible to reduce irritation of the blood vessel wall, even without special provision of a separate member made of a low-hardness material at the very tip end of the catheter main body 11. Furthermore, there is also less risk of detachment which may occur when a separate member is provided, and it is also possible to avoid increasing the number of components.

Moreover, the embodiment may be modified in the following manner.

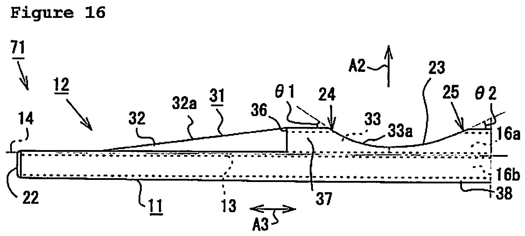

In the above embodiment, an example was described in which the angles θ1, θ2 were equal, but these angles θ1, θ2 do not necessarily have to be equal. For example, with the dialysis catheter 71 according to a different embodiment shown in Figure 16, the angle θ1 is set at approximately 33°, while the angle θ2 is set to be a smaller value (approximately 28°). In other words, the angles θ1, θ2 may be set at any value, provided that it is within the preferred range of 35° or less.

In the embodiment described above, the catheter main body 11 which was employed had a two-layer structure comprising the relatively soft resin layer 41 and the relatively hard resin layer 42, but this is not essential, so it is of course equally possible to employ a catheter main body having a single-layer structure.

In the embodiment described above, polyurethane resin was selected as the resin material for forming the soft resin layer 41 and the hard resin layer 42, but another flexible, elastic resin material may equally be selected, such as polyethylene, polypropylene, polyamide, polyvinyl chloride, silicone, or polyether-block-amide copolymer, for example. Furthermore, this was also stated in the above embodiment, but if any of these resins is selected as the resin material which forms the soft resin layer 41 and hard resin layer 42, the same resin is preferably selected as the resin material which forms the closure member 31.

In the embodiment described above, the blood removal holes 23, 64 and the blood feed hole 63, constituting side holes, had a shape in which the side wall 37 forming the second lumen was cut away in the shape of an arc, when viewed from the first direction A1, but this shape is not limited to such a shape, and a shape which is cut away in the shape of a flat isosceles triangle is equally feasible.

## Claims

1. A dialysis catheter (1; 61) comprising:
a catheter main body (11) having a first lumen (16b; 66c) which communicates with a tip-end hole (22; 62) defining a tip-end opening (36) that opens at a tip end of the catheter main body (11), and a second lumen (16a; 66b) which communicates with a side hole (23; 63, 64) that opens in a region of the tip end of the catheter main body (11), wherein:
the side hole (23; 63, 64) is a long hole extending in the catheter long-axis (14) direction (D3) which is formed by cutting away a side wall (37) of the catheter main body (11) forming the second lumen (16a; 66b), wherein a length of a major axis (d1) of the side hole (23; 63, 64) is at least 1.5 times an outer diameter (D1) of the catheter main body (11) at a point where the side hole (23; 63, 64) is formed, and a length of a minor axis (d2) of the side hole (23; 63, 64) is between 0.5 and 0.9 times the outer diameter (D1) of the catheter main body (11);
at a point where the side hole (23; 63, 64) is formed, when viewed from a first direction (A1) which is orthogonal to an opening direction (A2) of the side hole (23; 63, 64) and orthogonal to the catheter long-axis (14) direction (D3), an angle (θ1) formed by a tip-end opening edge (24) of the side hole (23; 63, 64) and a catheter long-axis (14) direction (D3), and an angle (θ2) formed by a base-end opening edge (25) of the side hole (23; 63, 64) and the catheter long-axis (14) direction (D3) are both 35° or less; and **characterised in that**
the catheter main body also has a closure member (31; 31A) having a tip-end side 32 having a shape with an outermost tapered surface 32a which is inclined with a reduced diameter toward the tip end for insertion into a blood vessel, and an insertion part 34 for insertion into the tip-end opening (36) of the second lumen (16a; 66b), the insertion part 34 having a base-end side (33) that extends to a position to seal the tip-end region of the side hole (23; 63, 64), and when viewed from the first direction (Al), a base-end surface (33a) of the closure member (31; 31A) is shaped to follow the shape of the tip-end opening edge (24) of the side hole (23; 63, 64).

2. A dialysis catheter (1; 61) according to Claim 1, **characterized in that** the side hole (23; 63, 64) has a shape, when viewed from the first direction (Al), in which the side wall (37) forming the second lumen (16a; 66b) has been cut away in the shape of an arc, and the base-end surface (33a) of the closure member (31; 31A) forms a concave curved surface.

3. A dialysis catheter (1; 61) according to Claim 1 or 2, **characterized in that** the catheter main body (11) has a hardness gradient so as to become softer from a base-end side of the catheter main body (11) towards a tip-end side of the catheter main body (11).

4. A dialysis catheter (1; 61) according to Claim 3, **characterized in that** the side wall (38) of the catheter main body (11) forming the first lumen (16b; 66c) and the side wall (37) forming the second lumen (16a; 66b) have a two-layer structure comprising a soft resin layer (41) which is relatively flexible and a hard resin layer (42) which is relatively rigid, and the proportion of the catheter main body (11) occupied by the soft resin (41) layer increases from the base-end side of the catheter main body (11) towards the tip-end side of the catheter main body (11), while a dividing wall (13) which partitions the catheter main body (11) into the first lumen 16b; 66c) and the second lumen (16a; 66b) comprises only the hard resin layer (42).

5. A dialysis catheter (1) according to Claims 1 to 4, **characterized in that** the catheter (1) is a double-lumen catheter comprising a blood feed lumen serving as the first lumen (16b) and a blood removal lumen serving as the second lumen (16a); and
the tip-end hole (22) is a blood feed hole, the side hole (23) is a blood removal hole, a tip-end side of the closure member (31) closes off the tip-end opening (36) of the blood removal lumen (16a), and the base-end side of the closure member (31) seals the tip-end region of the blood removal hole (23).

6. A dialysis catheter (61) according to Claims 1 to 4, **characterized in that** the catheter (61) is a triple-lumen catheter comprising a transfusion lumen serving as the first lumen (66c), a blood feed lumen serving as the second lumen (66b) and a blood removal lumen serving as the third lumen (66a); and
the tip-end hole is a transfusion hole (62), the side hole positioned at the tip-end side is a blood feed hole (63), the side hole positioned at the base-end side is a blood removal hole (64), the tip-end side of the closure member (31A) closes off the tip-end opening of the blood feed lumen (66b) and the tip-end opening of the blood removal lumen (66a), and the base-end side of the closure member (31A) seals the tip-end region of the blood feed hole (63) and the tip-end region of the blood removal hole (64).

## Patentansprüche

1. Dialysekatheter (1; 61), umfassend:
einen Katheter-Hauptkörper (11) mit einem ersten Lumen (16b; 66c), das mit einem Spitzen-Endloch (22; 62) verbunden ist, das eine Spitzen-Endöffnung (36) definiert, die sich an dem Spitzenende des Katheter-Hauptkörpers (11) öffnet, und einem zweiten Lumen (16a; 66b), das mit einem Seitenloch (23; 63; 64) verbunden ist, das sich in einem Bereich des Spitzenendes des Katheter-Hauptkörpers (11) öffnet, wobei:
das Seitenloch (23; 63; 64) ein langes Loch ist, das sich in der Richtung (D3) der Katheter-Längsachse (14) erstreckt und das durch Wegschneiden einer Seitenwand (37) des Katheter-Hauptkörpers (11), die das zweite Lumen (16a; 66b) bildet, gebildet ist, wobei die Länge der Hauptachse (d1) des Seitenlochs (23; 63; 64) wenigstens 1,5-mal dem Außendurchmesser (D1) des Katheter-Hauptkörpers (11) an einem Punkt, bei dem das Seitenloch (23; 63; 64) gebildet ist, beträgt und die Länge der kurzen Achse (d2) des Seitenlochs (23; 63; 64) zwischen 0,5 und 0,9-mal dem Außendurchmesser (D1) des Katheter-Hauptkörpers (11) beträgt;
an einem Punkt, an dem das Seitenloch (23; 63; 64), bei Ansicht aus einer ersten Richtung (A1) gebildet ist, die senkrecht zu der Öffnungsrichtung (A2) des Seitenlochs (23; 63; 64) und senkrecht zu der Richtung (D3) der Katheter-Längsachse (14) steht, der Winkel (θ1), der von dem Spitzenende-Öffnungsrand (24) des Seitenlochs (23; 63; 64) und der Richtung (D3) der Katheter-Längsachse (14) gebildet wird, und der Winkel (θ2), der von dem Basisende-Öffnungsrand (25) des Seitenlochs (23; 63; 64) und der Richtung (D3) der Katheter-Längsachse (14) gebildet wird, beide 35° oder weniger betragen; und **dadurch gekennzeichnet, dass**
der Katheter-Hauptkörper ferner ein Verschlusselement (31; 31a) aufweist, das eine Spitzenende-Seite (32) aufweist, die eine Form mit einer außenliegenden, sich verjüngenden Oberfläche (32a) aufweist, die mit einem verringerten Durchmesser zu dem Spitzenende zum Einführen in ein Blutgefäß geneigt ist, und einen Einführteil (34) zum Einführen in die Spitzenende-Öffnung (36) des zweiten Lumens (16a; 66b), wobei der Einführteil (34) eine Basisende-Seite (33) aufweist, die sich zu einer Position zum Abdichten des Spitzenende-Bereichs des Seitenlochs (23; 63; 64) erstreckt, und wenn aus der ersten Richtung (A1) gesehen, eine Basisende-Oberfläche (33a) des Verschlusselements (31; 31A) geformt ist, um der Form des Spitzenende-Öffnungsrands (24) des Seitenlochs (23; 63; 64) zu folgen.

2. Dialysekatheter (1; 61) gemäß Anspruch 1, **dadurch gekennzeichnet, dass** das Seitenloch (23; 63; 64) eine Form aufweist, wenn aus der ersten Richtung (A1) gesehen, bei der die Seitenwand (37), die das zweite Lumen (16a, 66b) bildet, in der Form eines Bogens weggeschnitten worden ist, und die Basisende-Oberfläche (33a) des Verschlusselements (31; 31A) eine konkave gekrümmte Oberfläche bildet.

3. Dialysekatheter (1; 61) gemäß Anspruch 1 oder 2, **dadurch gekennzeichnet, dass** der Katheter-Hauptkörper (11) einen Härtegradienten aufweist, um von der Basisende-Seite des Katheter-Hauptkörpers (11) in Richtung auf die Spitzenende-Seite des Katheter-Hauptkörpers (11) weicher zu werden.

4. Dialysekatheter (1; 61) gemäß Anspruch 3, **dadurch gekennzeichnet, dass** die Seitenwand (38) des Katheter-Hauptkörpers (11), die das erste Lumen (16b, 66c) bildet, und die Seitenwand (37), die das zweite Lumen (16a, 66b) bildet, eine zweischichtige Struktur aufweisen, die eine weiche Harzschicht (41), die vergleichsweise flexibel ist, und eine harte Harzschicht (42), die vergleichsweise starr ist, umfasst, und der Anteil des Katheter-Hauptkörpers (11), der von der weichen Harzschicht (41) eingenommen wird, von der Basisende-Seite des Katheter-Hauptkörpers (11) in Richtung auf die Spitzenende-Seite des Katheter-Hauptkörpers (11) zunimmt, während eine Trennwand (13), die den Katheter-Hauptkörper (11) in das erste Lumen (16b, 66c) und das zweite Lumen (16a, 66b) teilt, nur das harte Harz (42) umfasst.

5. Dialysekatheter (1; 61) gemäß Ansprüchen 1 bis 4, **dadurch gekennzeichnet, dass** der Katheter (1) ein Doppellumenkatheter ist, der ein Blutzufuhrlumen, das als das erste Lumen (16b) dient, und ein Blutentfernungslumen, das als das zweite Lumen (16a) dient, umfasst; und
das Spitzenende-Loch (22) ein Blutzufuhrloch ist, das Seitenloch (23) ein Blutentfernungsloch ist, die Spitzenende-Seite des Verschlusselements (31) die Spitzenende-Öffnung (36) des Blutentfernungslumens (16a) abschließt und die Basisende-Seite des Verschlusselements (31) den Spitzenende-Bereich des Blutentfernungslochs (23) abdichtet.

6. Dialysekatheter (1; 61) gemäß Ansprüchen 1 bis 4, **dadurch gekennzeichnet, dass** der Katheter (61) ein Dreilumenkatheter ist, der ein Transfusionslumen, das als das erste Lumen (66c) dient, ein Blutzufuhrlumen, das als das zweite Lumen (66b) dient, und ein Blutentfernungslumen, das als das dritte Lumen (66a) dient, umfasst; und
das Spitzenende-Loch ein Transfusionsloch (62) ist, das Seitenloch an der Spitzenende-Seite angeordnet ein Blutzufuhrloch (63) ist, das Seitenloch an der Basisende-Seite angeordnet ein Blutentfernungsloch (64) ist, die Spitzenende-Seite des Verschlusselements (31A) die Spitzenende-Öffnung des Blutzufuhrlumens (66b) und die Spitzenende-Öffnung des Blutentfernungslumens (66a) abschließt und die Basisende-Seite des Verschlusselements (31A) den Spitzenende-Bereich des Blutzufuhrlochs (63) und den Spitzenende-Bereich des Blutentfernungslochs (64) abdichtet.

## Revendications

1. Cathéter de dialyse (1 ; 61) comprenant :
un corps principal de cathéter (11) ayant une première lumière (16b ; 66c) qui communique avec un trou d'extrémité de pointe (22 ; 62) définissant une ouverture d'extrémité de pointe (36) qui s'ouvre au niveau d'une extrémité de pointe du corps principal de cathéter (11), et une seconde lumière (16a ; 66b) qui communique avec un trou latéral (23 ; 63, 64) qui s'ouvre dans une région de l'extrémité de pointe du corps principal de cathéter (11), dans lequel :
le trou latéral (23 ; 63, 64) est un trou long s'étendant dans la direction (D3) de l'axe long (14) du cathéter qui est formé en découpant une paroi latérale (37) du corps principal de cathéter (11) formant la seconde lumière (16a ; 66b), où une longueur d'un axe majeur (d1) du trou latéral (23 ; 63, 64) représente au moins 1,5 fois un diamètre externe (D1) du corps principal de cathéter (11) au niveau d'un point où le trou latéral (23 ; 63, 64) est formé, et une longueur d'un axe mineur (d2) du trou latéral (23 ; 63, 64) représente entre 0,5 et 0,9 fois le diamètre externe (D1) du corps principal de cathéter (11) ;
au niveau d'un point où le trou latéral (23 ; 63, 64) est formé, lorsqu'on observe à partir d'une première direction (A1) qui est orthogonale à une direction d'ouverture (A2) du trou latéral (23 ; 63, 64) et orthogonale à la direction (D3) de l'axe long (14) du cathéter, un angle (θ1) formé par un bord d'ouverture d'extrémité de pointe (24) du trou latéral (23 ; 63, 64) et une direction (D3) de l'axe long (14) du cathéter, et un angle (θ2) formé par un bord d'ouverture d'extrémité de base (25) du trou latéral (23 ; 63, 64) et la direction (D3) de l'axe long (14) du cathéter sont tous deux de 35° ou inférieur ; et **caractérisé en ce que** :
le corps principal de cathéter a un élément de fermeture (31 ; 31A) ayant un côté d'extrémité de pointe (32) ayant une forme avec la surface progressivement rétrécie située le plus à l'extérieur (32a) qui est inclinée avec un diamètre réduit vers l'extrémité de pointe pour l'insertion dans un vaisseau sanguin, et une partie d'insertion (34) pour l'insertion dans l'ouverture d'extrémité de pointe (36) de la seconde lumière (16a ; 66b), la partie d'insertion (34) ayant un côté d'extrémité de base (33) qui s'étend dans une position pour sceller la région d'extrémité de pointe du trou latéral (23 ; 63, 64), et lorsqu'elle est observée depuis la première direction (A1), une surface d'extrémité de base (33a) de l'élément de fermeture (31 ; 31A) est formée pour suivre la forme du bord d'ouverture d'extrémité de pointe (24) du trou latéral (23 ; 63, 64).

2. Cathéter de dialyse (1 ; 61) selon la revendication 1, **caractérisé en ce que** le trou latéral (23 ; 63, 64) a une forme, lorsqu'il est observé depuis la première direction (A1), dans laquelle la paroi latérale (37) formant la seconde lumière (16a ; 66b) a été découpée selon la forme d'un arc, et la surface d'extrémité de base (33a) de l'élément de fermeture (31 ; 31A) forme une surface incurvée concave.

3. Cathéter de dialyse (1 ; 61) selon la revendication 1 ou 2, **caractérisé en ce que** le corps principal de cathéter (11) a un gradient de dureté afin de devenir plus souple qu'un côté d'extrémité de base du corps principal de cathéter (11) vers un côté d'extrémité de pointe du corps principal de cathéter (11).

4. Cathéter de dialyse (1 ; 61) selon la revendication 3, **caractérisé en ce que** la partie latérale (38) du corps principal de cathéter (11) formant la première lumière (16b ; 66c) et la paroi latérale (37) formant la seconde lumière (16a ; 66b) ont une structure à deux couches comprenant une couche de résine souple (41) qui est relativement flexible et une couche de résine dure (42) qui est relativement rigide, et la proportion du corps principal de cathéter (11) occupée par la couche de résine souple (41) augmente du côté d'extrémité de base du corps principal de cathéter (11) vers le côté d'extrémité de pointe du corps principal de cathéter (11), alors qu'une paroi de division (13) qui sépare le corps principal de cathéter (11) dans la première lumière (16b ; 66c) et la seconde lumière (16a ; 66b) comprend uniquement la couche de résine dure (42).

5. Cathéter de dialyse (1) selon les revendications 1 à 4, le cathéter (1) étant **caractérisé en ce qu'**il est un cathéter à double lumière comprenant une lumière d'alimentation de sang servant de première lumière (16b) et une lumière d'extraction de sang servant de seconde lumière (16a) ; et
le trou d'extrémité de pointe (22) est un trou d'alimentation de sang, le trou latéral (23) est un trou d'extraction de sang, un côté d'extrémité de pointe de l'élément de fermeture (31) ferme l'ouverture d'extrémité de pointe (36) de la lumière d'extraction de sang (16a), et le côté d'extrémité de base de l'élément de fermeture (31) scelle la région d'extrémité de pointe du trou d'extraction de sang (23).

6. Cathéter de dialyse (61) selon les revendications 1 à 4, le cathéter (61) étant **caractérisé en ce qu'**il est un cathéter à triple lumière comprenant une lumière de transfusion servant de première lumière (66c), une lumière d'alimentation de sang servant de deuxième lumière (66b) et une lumière d'extraction de sang servant de troisième lumière (66a) ; et
le trou d'extrémité de pointe est un trou de transfusion (62), le trou latéral positionné au niveau du côté d'extrémité de pointe est un trou d'alimentation de sang (63), le trou latéral positionné au niveau du côté d'extrémité de base est un trou d'extraction de sang (64), le côté d'extrémité de pointe de l'élément de fermeture (31A) ferme l'ouverture d'extrémité de pointe de la lumière d'alimentation de sang (66b) et l'ouverture d'extrémité de pointe de la lumière d'extraction de sang (66a), et le côté d'extrémité de base de l'élément de fermeture (31A) scelle la région d'extrémité de pointe du trou d'alimentation de sang (63) et la région d'extrémité de pointe du trou d'extraction de sang (64).
